# EUROPEAN PATENT SPECIFICATION

(11) **EP 3 015 112 B1**
(45) Date of publication and mention of the grant of the patent: **18.07.2018**
(21) Application number: 14865261.3
(22) Date of filing: 25.11.2014
(51) Int. Cl.: A61K 36/899, A61K 36/483, A61K 36/482, A61K 36/79, A61K 36/46, A61K 36/232, A61K 36/65, A61K 36/489, A61K 36/748, A61K 36/488, A61K 36/484, A61K 36/48, A61K 36/36, A61K 36/258, A61K 35/36, A61K 9/08, A61P 1/16, A61P 7/10, A61K 9/00

(54) **MEDICAMENT FOR TREATMENT OF HEPATIC ASCITES AND PREPARATION METHOD THEREFOR**
ARZNEIMITTEL ZUR BEHANDLUNG VON HEPATISCHEM ASZITES UND HERSTELLUNGSVERFAHREN DAFÜR
MÉDICAMENT POUR LE TRAITEMENT DE L'ASCITE HÉPATIQUE, ET SON PROCÉDÉ DE PRÉPARATION

(30) Priority: 27.11.2013 CN 201310610469
(43) Date of publication of application: 04.05.2016
(73) Proprietor: Liu, Mingjie, Weifang, Shandong 261041 (CN)
(72) Inventor: Liu, Mingjie, Weifang, Shandong 261041 (CN)
(74) Representative: Huang, Liwei
(86) International application number: PCT/CN2014/092159
(87) International publication number: WO 2015/078356

(56) References cited:
- CN-A- 1 762 484
- CN-A- 101 062 368
- CN-A- 101 816 745
- CN-A- 101 897 908
- CN-A- 101 897 908
- CN-A- 101 940 759
- CN-A- 101 940 759
- CN-A- 102 342 995
- CN-A- 102 488 850
- CN-A- 102 895 547
- CN-A- 103 212 033
- CN-A- 103 656 277

## Description

### TECHNICAL FIELD

The present invention relates to a traditional Chinese medicine and a preparation method thereof, and particularly relates to a medicament for treating hepatic ascites and a preparation method thereof.

### BACKGROUND

Ascites due to cirrhosis is colloquially referred to as hepatic ascites. A small amount (typically about 50 ml) of free ascites exists in the abdominal cavity of a normal human, which plays a role of maintaining lubrication among organs, however, when excess free fluids occur in the abdominal cavity, they are called ascites. The ascites due to cirrhosis is a chronic liver disease. Bulk, nodular and diffuse hepatocellular changes, necrosis and regeneration as well as regeneration and necrosis assist tissue fibroplasia and scar contraction to cause a result that the liver becomes harder to develop liver cirrhosis. Hypohepatia caused by liver cirrhosis causes portal hypertension to result in splenomegaly, and a protein fluid leaking due to non-absorption of protein and vitamins forms an ascites syndrome.

Hepatic ascites is a prominent clinical manifestation of the liver cirrhosis; more than 75 % of patients with decompensated liver cirrhosis suffer from ascites. A mechanisms formed by ascites is excess retention of sodium and water, and is related to the following local factors and systemic factors of the abdominal cavity: 1. increased portal venous pressure: when it exceeds 300 mmH2O, a hydrostatic pressure of a vascular bed of organs in the abdominal cavity increases, resorption of an interstitial fluid is reduced to leak into the abdominal cavity. 2. Hypoalbuminemia: when the content of albumin is less than 31 g/L, a plasma colloid osmotic pressure decreases, which causes exosmosis of blood compositions. 3. Excessively generated lymph: when liver venous reflux is obstructed, blood permeates to parasinoidal spaces from hepatic sinusoidal walls, which causes a result that a generation amount of gallbladder lymph increases, and exceeds the ability of ductus thoracicus drainage, and lymph permeates to the abdominal from Glisson's capsule and porta hepatic lymph vessels. 4. Increased kidney sodium reabsorption caused by increased secondary aldosterone. 5. Increased water reabsorption caused by increased antidiuretic hormone secretion. 6. Insufficient effective circulating blood volume: sympathetic activities increase, and prostaglandin, atrium and kallikrein-plasmakinin activities decrease, so that renal blood flow, sodium excretion and urine output decrease. The above multiple factors act in ascites formation and continued stages to some extent, wherein hypohepatia and portal hypertension run through the whole process. The ascites is associated with abdominal distension frequently before developing, a large amount of ascites causes the belly to bulge and the abdominal wall to tighten and highlight (like a frog belly), the patients walk difficultly, sometime, the diaphragm remarkably elevates, and the breathing and the umbilical hernia appear. Partial patients are companied by hydrothorax, which are often rich in the right side due to the leakage of the ascites into the thoracic cavity through the diaphragmatic lymphatic vessel or through the valvular opening.

Hepatic ascites is an ending stage of liver cirrhosis, which is also a watershed of compensated liver cirrhosis and decompensated liver cirrhosis; once the ascites occurs, it indicates that the function of the liver self is very poor already. Reasons developing the hepatic ascites are liver-derived, kidney-derived, autoimmune and the like. At present, no drugs with exact treatment effects are available in western medicines for treating the hepatic ascites, great side effects may be caused when the hepatic ascites is treated, drugs in western medicine replenishe fluids, resist infection, protect the liver and induce diuresis, but usually hurt the kidney, and may not achieve integral regulation of viscera, thereby making a condition further develop to hepatorenal syndrome, uremia and kidney failure. Although the fluids are replenished for the hepatic ascites, a diuretic is also utilized but directly hurts the kidney, accordingly, this is the greatest defect of the western medicine when being used for treating the hepatic ascites. The western medicine therapy is large in treatment side effects. Puncture therapy employed clinically is high in relapse, brings pain to the patients, and easily induces the infection. Large-dose usage of injectio mannitou and furosemidum easily induce electrolyte disturbance.

Because ascites pathogenesis is complex, symptoms caused by deficiency and excess as well as chills and fever are different, and conditions vary, the traditional Chinese medicine employs a fixed prescription to generally difficultly satisfy clinical requirements, many people creatively employed a method of differentiation of symptoms and signs for classification of syndromes, and proposed seven therapeutic methods for treating swelling and eight therapeutic methods for treating swelling. The fixed prescription was employed in the earlier Chinese medicine, individual herbs are, for example, euphorbia kansui, croton seed, daphne genkwa, radix phytolaccae, euphorbia pekinensis, pharbitis seed and the like; because fluids were drained mainly through the intestinal tract, diuresis was not induced remarkably; because a reaction is strong, electrolyte disturbance is easily caused, and few people employ this method now.

### SUMMARY

An objective of the present invention is to provide a medicament for treating hepatic ascites, with functions of protecting liver, relieving internal heat, cleansing blood and benefiting vital energy, and a preparation method thereof.

The medicament for treating the hepatic ascites, provided by the present invention, comprises the following components in parts by weight: 15-55 parts of shiraia bambusicola, 15-55 parts of spina gleditsiae, 15-25 parts of cornu bubali, 15-45 parts of caulis bambusae in taeniam, 15-25 parts of angelica pubescens, 15-25 parts of fructus schizandrae, 15-25 parts of radix puerariae, 15-75 parts of hedyotis diffusa, 5-15 parts of glycyrrhiza uralensis, 15-55 parts of lotus leaf, 5-35 parts of semen cassiae, 5-15 parts of radix pseudostellariae, 5-15 parts of American ginseng, 5-35 parts of cortex eucommiae, 5-25 parts of radix paeoniae alba and 5-35 parts of sophora flavescens.

As a further improvement of the present invention, parts by weight of components are as follows: 35-55 parts of shiraia bambusicola, 35-55 parts of spina gleditsiae, 15-25 parts of cornu bubali, 15-25 parts of caulis bambusae in taeniam, 15-25 parts of angelica pubescens, 15-25 parts of fructus schizandrae, 15-25 parts of radix puerariae, 35-55 parts of hedyotis diffusa, 5-15 parts of glycyrrhiza uralensis, 35-55 parts of lotus leaf, 5-15 parts of semen cassiae, 5-15 parts of radix pseudostellariae, 5-15 parts of American ginseng, 5-15 parts of cortex eucommiae, 5-15 parts of radix paeoniae alba and 5-15 parts of sophora flavescens.

As a further improvement of the present invention, parts by weight of components are as follows: 15-55 parts of shiraia bambusicola, 15-25 parts of spina gleditsiae, 15-25 parts of cornu bubali, 35-45 parts of caulis bambusae in taeniam, 15-25 parts of angelica pubescens, 15-25 parts of fructus schizandrae, 15-25 parts of radix puerariae, 15-25 parts of hedyotis diffusa, 5-15 parts of glycyrrhiza uralensis, 5-15 parts of lotus leaf, 25-35 parts of semen cassiae, 5-15 parts of radix pseudostellariae, 5-15 parts of American ginseng, 5-15 parts of cortex eucommiae, 15-25 parts of radix paeoniae alba and 5-15 parts of sophora flavescens.

A preparation method of the above medicament for treating the hepatic ascites comprises the following steps performed sequentially:
(A) putting shiraia bambusicola, spina gleditsiae, semen cassiae, fructus schizandrae, cortex eucommiae, radix paeoniae alba, angelica pubescens, sophora flavescens, radix puerariae, hedyotis diffusa and caulis bambusae in taeniam in 1000-2000 parts by weight of clear water with a temperature of 20-30 °C according to the above parts by weight and soaking for 4-6 h, putting in an earthen pot and boiling, then keeping a boiling state and decocting for 20-30 min, then adding glycyrrhiza uralensis and lotus leaf in proportion, continuously decocting for 25-35 min, removing herb residues by filtration after naturally cooling to obtain a decoction I; respectively soaking radix pseudostellariae, American ginseng and cornu bubali with 100 parts by weight of clear water with a temperature of 20-30 °C for 4-6 h, then putting the radix pseudostellariae, the American ginseng, the cornu bubali and a soaking solution in the earthen pot and boiling, then keeping a boiling state for 30-50 min, removing herb residues after naturally cooling to obtain a decoction II;
(B) mixing the decoction I with the decoction II, heating to boiling and keeping for 5 min, and naturally cooling to obtain the medicament for treating hepatic ascites.

Shiraia bambusicola is also called tabasheer, nandina flower, henon bamboo flower and the like, and belongs to fungi, ascomycotion, pyrenomycetes, hypocreales, hypocreaceae, and shiraia fungi. It flourishes in a decaying or to-be-decayed bamboo forest, and medicinal parts of the shiraia bambusicola refer to stromas of fungus shiraia bambusicola. It has functions of resolving phlegm for relieving cough, regulating qi to alleviate pain, and relieving convulsion.

Spina gleditsiae refers to thorns of gleditsia sinensis of a leguminous plant, and is warm-natured, is acrid in taste, and is slightly poisonous.

Cornu bubali refers to a substitute article of rhinoceros horn of a traditional Chinese medicine, and has functions of clearing heat, cooling blood, arresting convulsion and relieving internal heat.

Caulis bambusae in taeniam is also called bamboo skin, green caulis bambusae in taeniam and phyllostachys glauca shavings. It refers to dried middle layers of stalks of Bambusa tuldoides Munro, D.beecheyana var. pubescens(P.F. Li)Keng f or Phyllostachys glauca McClure of a gramineous plant. It contains lignin and cellulose. It is slightly cold-natured, and is sweet in taste. It is commonly used for treating cough due to lung heat, yellow phlegm, dysphoria caused by heat, and palpitation in traditional Chinese medicine.

Angelica pubescens refers to dried roots of Angelica biserrata (Shan et Yuan) Yuan et Shan of an umbelliferae plant, is pungent, bitter, and slightly warm-natured, and is used for treating anemofrigid-damp arthralgia, waist and knee ache, headache caused by invading a Shaoyin channel by wind, headache and dentalgia. Modern researches have found that, it has functions of resisting inflammation, alleviating pain, and tranquilizing mind, has a function of inhibiting platelet aggregation, and has an antihypertensive effect but is not persistent; bergapten, xanthotoxin and the like contained in the angelica pubescens have functions of photosensitization and tumor resistance.

Fructus schizandrae is also called Xuanji, Huiji, Wumeizi, zanthoxylum schinifollum, the fruit of Chinese magnolia vine, Schisandra chinensis (Turcz.) Baill and the like, and refers to mature fruits of Schisandra sphenanthera Rehd. et Wils. or Schisandra chinensis (Turcz.) Baill. of a plant. It has herbal functions of being tonic and strengthening the constitution, and protecting the liver.

Radix puerariae refers to dried roots of pueraria lobata ohwi of a leguminous plant. It has functions of antipyresis, anti-inflammation, antibiosis and immunization.

Hedyotis diffusa has a scientific name of Hedyotis diffusa, is a plant of hedyotis of rubiaceae, and is commonly used for treating dyspnea with cough due to lung heat, swollen sore throat, periappendicular abscess, furuncles, poisonous snake bite and the like.

Glycyrrhiza uralensis has a prescription name of Glycyrrhiza uralensis Fisch, Shengcao, radix glycyrrhizae, Jiucao, radix glycyrrhizae preparata, Caoshao, fine licorice root, Shengcao tip and the like, refers to root and rhizome of dicotyledons Leguminosae Glycyrrhiza uralensisFisch., G. inflataBat., or G. glabraL, and has major functions of clearing away heat and toxic materials, expelling phlegm to arrest coughing, relieving stomach duct and abdomen, and the like.

Lotus leaf has a prescription name of lotus leaf, folium nelumbinis, fresh lotus leaf, dried lotus leaf, lotus leaf charcoal and the like, refers to dried leaves of nelumbo nuficera gaertn of nymphaeaceae, and has functions of reliving summer heat and removing dampness, tonifying spleen and invigorating splenic yang, dissipating blood stasis and stopping bleeding.

Semen cassiae refers to dried mature seeds of Cassia obtusifolia L. or Cassia tora L of an annual herb of Leguminosae sp., is also called senna obtusifolia, horseshoe senna obtusifolia, false mung bean, and Jingudou, is bitter-sweet, slightly cold-natured and non-poisonous, and has functions of clearing heat and brightening eyes, and relaxing bowel.

Radix pseudostellariae is also called Haiershen and Tongshen refers to tuberous roots of Pseudostellaria heterophylla (Miq.) Pax of a perennial herb of caryophyllaceae, is sweet, slightly bitter, and slightly warm-natured, is a herb for restoring vital energy and a herb for tonifying qi, and has major functions of benefiting spleen and lung, and supplementing qi and promoting production of body fluid.

American ginseng refers to dried roots of Panax quinquefolium L of Araliaceae, is one kind of ginseng, and is also called Guangdong ginseng and panax quinquefolius.

Cortex eucommiae is also called Sijianshupi, Simianpi, Mianshupi and Jiaoshu, refers to dried barks of Eucommia ulmoides Oliver of eucommiaceae, is a rare medicinal herb in China, and has pharmacological functions of nourishing liver and kidney, strengthening bones and muscles, clearing body junk, strengthening substance metabolism of human cells, preventing muscle and bone from ageing, balancing human body blood pressure, decomposing cholesterol in vivo, reducing body fat, restoring vascular elasticity, inducing diuresis and clearing heat, being wide in antimicrobial spectrum, exciting nervous centralis, and improving leucocytes.

Radix paeoniae alba is also called white paeony root, Huazi, Paeonia lactiflora Pall., radix paeoniae rubra, Hangzhou paeonia albiflora, Dabaishao, and the like, refers to roots of Chinese herbaceous peony of ranunculaceae of magnoliopsida, has functions of protecting liver and reducing enzyme, and is commonly compatible with radix bupleuri, angelica sinensis, dried rehmannia root and the like.

Sophora flavescens has a scientific name of Sophora flavescens, is a plant of sophorae of leguminosae, and has major functions of clearing heat and drying dampness, killing insects and inducing diuresis.

The medicament for treating hepatic ascites, provided by the present invention, is prepared from principal herbs such as shiraia bambusicola, spina gleditsiae, cornu bubali, caulis bambusae in taeniam, angelica pubescens, fructus schizandrae, radix puerariae and hedyotis diffusa and secondary herbs such as glycyrrhiza uralensis, lotus leaf, semen cassiae, radix pseudostellariae, American ginseng, cortex eucommiae, radix paeoniae alba and sophora flavescens, and has comprehensive functions of protecting liver, relieving internal heat, cleansing blood, and benefiting vital energy. Based on treatment of differentiation of symptoms and signs for classification of syndromes, a treatment is administered according to syndromes, thereby improving a treatment effect. Improved treatment and recovery effects are provided for hepatic ascites caused by hepatitis B and liver cirrhosis.

### DETAILED DESCRIPTION

### Embodiment 1:

Preparing for herbs: preparing for the following components in parts by weight: 15-55 parts of shiraia bambusicola, 15-55 parts of spina gleditsiae, 15-25 parts of cornu bubali, 15-45 parts of caulis bambusae in taeniam, 15-25 parts of angelica pubescens, 15-25 parts of fructus schizandrae, 15-25 parts of radix puerariae, 15-75 parts of hedyotis diffusa, 5-15 parts of glycyrrhiza uralensis, 15-55 parts of lotus leaf, 5-35 parts of semen cassiae, 5-15 parts of radix pseudostellariae, 5-15 parts of American ginseng, 5-35 parts of cortex eucommiae, 5-25 parts of radix paeoniae alba and 5-35 parts of sophora flavescens. Particular contents of the components may be regulated within the above range according to characteristics of indications and sources of raw materials.

A preparation method comprises the following steps:
(A) putting shiraia bambusicola, spina gleditsiae, semen cassiae, fructus schizandrae, cortex eucommiae, radix paeoniae alba, angelica pubescens, sophora flavescens, radix puerariae, hedyotis diffusa and caulis bambusae in taeniam in 1000-2000 parts by weight of clear water with a temperature of 20-30 °C according to the above parts by weight and soaking for 4-6 h, putting in an earthen pot and boiling, then keeping a boiling state and decocting for 20-30 min, then adding glycyrrhiza uralensis and lotus leaf in proportion, continuously decocting for 25-35 min, removing herb residues by filtration after naturally cooling to obtain a decoction I; respectively soaking radix pseudostellariae, American ginseng and cornu bubali with 100 parts by weight of clear water with a temperature of 20-30 °C for 4-6 h, then putting the radix pseudostellariae, the American ginseng, the cornu bubali and a soaking solution in the earthen pot and boiling, then keeping a boiling state for 30-50 min, removing herb residues after naturally cooling to obtain a decoction II;
(B) mixing the decoction I with the decoction II, heating to boiling and keeping for 5 min, and naturally cooling to obtain the medicament for treating hepatic ascites.

A taking method is as follows: the above medicament for treating the hepatic ascites is divided into portions of 50 g each, and one portion is taken at one time, and three times a day. 15 days constitute one therapeutic course.

A patient, Wang XX, male, 49 years-old, came from Dezhou city in Shandong province, and suffered from hepatic ascites. The patient complained of intermittent gingival bleeding for more than 3 years and abdominal distension and pain for 2 months. A few years ago, the patient discovered that he had Hepatitis B, with HBsAg, HBeAg and HBcAb all of being tested to be positive in one medical examination, and then treated in a local hospital; and after several months, his liver functioned normally. After discharged, the patient often felt tired, especially was lack of power more seriously after hard work, and started suffering from abdominal distension and pain. Indicators were tested as follows:
Five serdogical markers of hepatitis B: HBsAg(+)HBeAg(+)HBcAb(+)
Liver function: ALT 150.9U/L AST 1875.02U/L
Color Doppler ultrasound: portal vein 1.3 cm, thickness of spleen 6.2 cm, splenic vein 1.3 cm, ascites 12.3 cm
Diagnosis result: hepatitis hepatocirrhosis, Hepatitis B, active, ascites.

According to an examination result and a condition of the patient, in a case of serious ascites, the patient was treated by employing the medicament of this embodiment. An examination result after three courses of treatment showed that HBV-DNA of the patient turned negative, e antigen turned negative, the liver was functioned normally, and the ascites disappeared. Indicators of the patient returned to a normal range, and the patient suffering from liver cirrhosis perked up, and was cured.

### Embodiment 2:

Preparing for herbs: preparing for the following components in parts by weight: 35-55 parts of shiraia bambusicola, 35-55 parts of spina gleditsiae, 15-25 parts of cornu bubali, 15-25 parts of caulis bambusae in taeniam, 15-25 parts of angelica pubescens, 15-25 parts of fructus schizandrae, 15-25 parts of radix puerariae, 35-55 parts of hedyotis diffusa, 5-15 parts of glycyrrhiza uralensis, 35-55 parts of lotus leaf, 5-15 parts of semen cassiae, 5-15 parts of radix pseudostellariae, 5-15 parts of American ginseng, 5-15 parts of cortex eucommiae, 5-15 parts of radix paeoniae alba and 5-15 parts of sophora flavescens. Particular contents of the components may be regulated within the above range according to characteristics of indications and sources of raw materials.

A preparation method is identical to that of the embodiment 1.

A taking method is as follows: the above medicament for treating the hepatic ascites is divided into portions of 50 g each, and one portion is taken at one time, and three times a day. 15 days constitute one therapeutic course.

A patient Tian XX, male, 39 years-old, discovered that he had hepatitis B and liver cirrhosis in hospital in May of 2012 due to hepatalgia and vomissement. A condition exacerbated after hard work, and then relapsed. Six months ago, these symptoms exacerbated in association with abdominal bulge, oliguria and edema of both lower extremity; and treatment effects in a local hospital were unsatisfactory.

Symptoms of the patient when arrived at the hospital were as follows: listlessness, emaciation, serious abdominal bulge, fist-sized and firm hepatomphalocele, positive ascites syndrome, and edema of lower extremity. B-mode ultrasound: uneven liver, portal hypertension, splenomegaly, and massive ascites. A liver function test was as follows: the liver was seriously damaged, and the content of albumin was 29 g.

In a case of the condition of the patient, hepatalgia of the patient relieved after being treated by employing the medicament of this embodiment for one week, and symptoms such as anepithymia, weakness, ascites, jaundice, and hemorrhagic tendency were remarkably alleviated. After one month, the content of albumin of the patient recovered to 50 g, and the patient was on recovery. After three months, the ascites subsided, and there was no edema in lower limbs. The medicament was stopped being used after treatment for a period of time, the patient was cured, and the condition was stable without relapse when the patient returned to the hospital for multiple times.

### Embodiment 3:

Preparing for herbs: preparing for the following components in parts by weight: 15-55 parts of shiraia bambusicola, 15-25 parts of spina gleditsiae, 15-25 parts of cornu bubali, 35-45 parts of caulis bambusae in taeniam, 15-25 parts of angelica pubescens, 15-25 parts of fructus schizandrae, 15-25 parts of radix puerariae, 15-25 parts of hedyotis diffusa, 5-15 parts of glycyrrhiza uralensis, 5-15 parts of lotus leaf, 25-35 parts of semen cassiae, 5-15 parts of radix pseudostellariae, 5-15 parts of American ginseng, 5-15 parts of cortex eucommiae, 15-25 parts of radix paeoniae alba and 5-15 parts of sophora flavescens. Particular contents of the components may be regulated within the above range according to characteristics of indications and sources of raw materials.

A preparation method is identical to that of the embodiment 1.

A taking method is as follows: the above medicament for treating the hepatic ascites is divided into portions of 50 g each, and one portion is taken at one time, and three times a day. 15 days constitute one therapeutic course.

A patient Cui XX, male, 45 years-old, came from Jinan city in Shandong province. Symptoms were as follows: edema of lower limbs, abdominal distention, poor appetite, scanty dark urine, regular bowel movements, pale complexion, cold limbs, pale tongue and white tongue coating, petechia in tongue, and deep thready pulse. A medical history was as follows: the patient presented sallow complexion, limb weakness, hepatalgia, weak breath and disinclination to talk in association with edema of lower limbs in May of 2009, and was hospitalized in a local hospital for four times due to hepatitis B, and was discharged after relieved. Examination results prior to admission were as follows: examination results: liver function: ALT 180.3u/L, AST 60.8u/L. B-mode ultrasound: hepatomegaly, inhomogenous internal echo, a large amount of opaque dark areas of fluid in the abdominal cavity, and light yellow viscous fluid produced in abdominocentesis to prompt ascites due to cirrhosis. Diagnosis result: liver cirrhosis in association with ascites.

After treatment to syndromes by employing the medicament of this embodiment for 10 days, edema of lower limbs of the patient was alleviated, urine volume gradually increased, complexion was slightly ruddy, and hepatalgia was alleviated. After one course of treatment, the edema substantially subsided. Reexamination results were as follows: liver function: ALT 40u/L, AST 35.1u/L. After continued treatment for one month, self symptoms of the patient disappeared, the edema of lower limbs subsided, the complexion was ruddy, hepatalgia disappeared, the liver functioned normally, B-mode ultrasound showed that there was no abnormality, and the patient was cured. After consolidation treatment for three months, the patient's condition did not relapse.

A patient, Li XX, 35 years-old, came from Tengzhou city. A medical history was as follows: the patient discovered that he had Hepatitis B, with HBsAg, HBeAg and HBcAb all of being tested to be positive in a medical examination of organize by his unit four years ago, then treated in a local country traditional Chinese hospital for half a month, and finally his liver functioned normally. The patient had abdominal distention and pain without precipitating factors recently within one month. After treatment as a "stomach illness" in a local clinic, the patient felt gradually aggravated abdominal distension, and developed edema of both lower extremity and increased frequency of bowel movements, and indicators were tested on admission as follows:
Five serdogical markers of hepatitis B: HBsAg(+)HBeAg(+)HBcAb(+)
Liver function : ALT: 157U/L AST: 178U/L
Color Doppler ultrasound: portal vein 1.3 cm, thickness of spleen 6.2 cm, splenic vein 1.1 cm, ascites 13.7 cm
Diagnosis result: hepatitis hepatocirrhosis, Hepatitis B, active, ascites.

By employing a medicament treatment solution of this embodiment, symptoms of the patient subjected to one course of treatment were remarkably improved. Effects in a second course of treatment were ideal. Reexamination results after three courses of treatment were as follows: HBV-DNA turned negative, e antigen turned negative, the liver was functioned normally, and the ascites disappeared. Indicators of the patient returned to a normal range, and the patient suffering from liver cirrhosis perked up, and was cured.

### Embodiment 4:

Preparing for herbs: preparing for the following components in parts by weight: 15 parts of shiraia bambusicola, 15 parts of spina gleditsiae, 15 parts of cornu bubali, 35 parts of caulis bambusae in taeniam, 15 parts of angelica pubescens, 15 parts of fructus schizandrae, 15 parts of radix puerariae, 15 parts of hedyotis diffusa, 5 parts of glycyrrhiza uralensis, 5 parts of lotus leaf, 25 parts of semen cassiae, 5 parts of radix pseudostellariae, 5 parts of American ginseng, 5 parts of cortex eucommiae, 15 parts of radix paeoniae alba and 5 parts of sophora flavescens.

A preparation method is identical to that of the embodiment 1.

### Embodiment 5:

Preparing for herbs: preparing for the following components in parts by weight: 55 parts of shiraia bambusicola, 25parts of spina gleditsiae, 25 parts of cornu bubali, 45 parts of caulis bambusae in taeniam, 25 parts of angelica pubescens, 25 parts of fructus schizandrae, 25 parts of radix puerariae, 25 parts of hedyotis diffusa, 15 parts of glycyrrhiza uralensis, 15 parts of lotus leaf, 35 parts of semen cassiae, 15 parts of radix pseudostellariae, 15 parts of American ginseng, 15 parts of cortex eucommiae, 25 parts of radix paeoniae alba and 15 parts of sophora flavescens.

A preparation method is identical to that of the embodiment 1.

According to clinical trial statistics, 50 patients suffering from hepatic ascites caused by various reasons are selected, and are treated by employing the medicament for treating the hepatic ascites, provided by the present invention, 41 patients were treated markedly effectively, 5 patients were treated effectively, and 4 patients were ineffectively treated, so that a clinical effective rate was 92 %.

## Claims

1. A medicament for treating hepatic ascites, comprising the following components in parts by weight: 15-55 parts of shiraia bambusicola, 15-55 parts of spina gleditsiae, 15-25 parts of cornu bubali, 15-45 parts of caulis bambusae in taeniam, 15-25 parts of angelica pubescens, 15-25 parts of fructus schizandrae, 15-25 parts of radix puerariae, 15-75 parts of hedyotis diffusa, 5-15 parts of glycyrrhiza uralensis, 15-55 parts of lotus leaf, 5-35 parts of semen cassiae, 5-15 parts of radix pseudostellariae, 5-15 parts of American ginseng, 5-35 parts of cortex eucommiae, 5-25 parts of radix paeoniae alba and 5-35 parts of sophora flavescens.

2. The medicament for treating hepatic ascites according to claim 1, wherein parts by weight of components are as follows: 35-55 parts of shiraia bambusicola, 35-55 parts of spina gleditsiae, 15-25 parts of cornu bubali, 15-25 parts of caulis bambusae in taeniam, 15-25 parts of angelica pubescens, 15-25 parts of fructus schizandrae, 15-25 parts of radix puerariae, 35-55 parts of hedyotis diffusa, 5-15 parts of glycyrrhiza uralensis, 35-55 parts of lotus leaf, 5-15 parts of semen cassiae, 5-15 parts of radix pseudostellariae, 5-15 parts of American ginseng, 5-15 parts of cortex eucommiae, 5-15 parts of radix paeoniae alba and 5-15 parts of sophora flavescens.

3. The medicament for treating hepatic ascites according to claim 1, wherein parts by weight of components are as follows: 15-55 parts of shiraia bambusicola, 15-25 parts of spina gleditsiae, 15-25 parts of cornu bubali, 35-45 parts of caulis bambusae in taeniam, 15-25 parts of angelica pubescens, 15-25 parts of fructus schizandrae, 15-25 parts of radix puerariae, 15-25 parts of hedyotis diffusa, 5-15 parts of glycyrrhiza uralensis, 5-15 parts of lotus leaf, 25-35 parts of semen cassiae, 5-15 parts of radix pseudostellariae, 5-15 parts of American ginseng, 5-15 parts of cortex eucommiae, 15-25 parts of radix paeoniae alba and 5-15 parts of sophora flavescens.

4. A preparation method of the medicament for treating hepatic ascites according to claim 1 or 2 or 3, wherein said method comprises the following steps performed sequentially:
(A) putting shiraia bambusicola, spina gleditsiae, semen cassiae, fructus schizandrae, cortex eucommiae, radix paeoniae alba, angelica pubescens, sophora flavescens, radix puerariae, hedyotis diffusa and caulis bambusae in taeniam in 1000-2000 parts by weight of clear water with a temperature of 20-30 °C according to the above parts by weight and soaking for 4-6 h, putting in an earthen pot and boiling, then keeping a boiling state and decocting for 20-30 min, then adding glycyrrhiza uralensis and lotus leaf in proportion, continuously decocting for 25-35 min, removing herb residues by filtration after naturally cooling to obtain a decoction I; respectively soaking radix pseudostellariae, American ginseng and cornu bubali with 100 parts by weight of clear water with a temperature of 20-30 °C for 4-6 h, then putting the radix pseudostellariae, the American ginseng, the cornu bubali and a soaking solution in the earthen pot and boiling, then keeping a boiling state for 30-50 min, removing herb residues after naturally cooling to obtain a decoction II;
(B) mixing the decoction I with the decoction II, heating to boiling and keeping for 5 min, and naturally cooling to obtain the medicament for treating hepatic ascites.

## Patentansprüche

1. Medikamente zur Behandlung von Leberaszites, **dadurch gekennzeichnet, dass** es folgende Bestandteilen in Gewichtsteilen enthält: Tabasheer 15-55, Saponin Thorn 15-55, Hörner von Buffalo 15-25, Caulis bambusae in Taeniis 15-45, Angelica pubescens 15-25, Früchte des Chinesischen Spaltkölbchens 15-25, Wurzel der Kudzu-Rebe 15-25, Hedyotis diffusa15-75, Lakritze 5-15, Lotosblatt 15-55, Cassia obtusifolia 5-35, Pseudostellaria 5-15, Fünfblättriger Ginseng 5-15, Eucommia ulmoides 5-35, Paeonia lactiflora 5-25, Sophora flavescens var. flavescens 5-35.

2. Medikamente zur Behandlung von Leberaszites nach Anspruch 1, **dadurch gekennzeichnet, dass** die Gewichtsanteile seiner Bestandteile wie folgt sind:
Tabasheer 35-55, Saponin Thorn 35-55, Hörner von Buffalo 15-25, Caulis bambusae in Taeniis 15-25, Angelica pubescens 15-25, Früchte des Chinesischen Spaltkölbchens 15-25, Wurzel der Kudzu-Rebe 15-25, Hedyotis diffusa35-55, Lakritze 5-15, Lotosblatt 35-55, Cassia obtusifolia 5-15, Pseudostellaria 5-15, Fünfblättriger Ginseng 5-15, Eucommia ulmoides 5-15, Paeonia lactiflora 5-15, Sophora flavescens var. flavescens5-15.

3. Medikamente zur Behandlung von Leberaszites nach Anspruch 1, **dadurch gekennzeichnet, dass** die Gewichtsanteile seiner Bestandteile wie folgt sind:
Tabasheer 15-55, Saponin Thorn 15-25, Hörner von Buffalo 15-25, Caulis bambusae in Taeniis 35-45, Angelica pubescens 15-25, Früchte des Chinesischen Spaltkölbchens 15-25, Wurzel der Kudzu-Rebe 15-25, Hedyotis diffusa15-25, Lakritze 5-15, Lotosblatt 5-15, Cassia obtusifolia 25-35, Pseudostellaria 5-15, Fünfblättriger Ginseng 5-15, Eucommia ulmoides 5-15, Paeonia lactiflora 15-25, Sophora flavescens var. flavescens 5-15.

4. Methode zur Vorbereitung der Medikation zur Behandlung von Leberaszites nach Anspruch 1 oder 2 oder 3, **dadurch gekennzeichnet, dass** es folgende Schritte in der Reihenfolge enthält:
A) die Tabasheer, Saponin Thorn, Cassia obtusifolia, Früchte des Chinesischen Spaltkölbchens, Eucommia ulmoides, Paeonia lactiflora, Angelica pubescens, Sophora flavescens var. flavescens, Wurzel der Kudzu-Rebe, Hedyotis diffusa und Caulis bambusae in Taeniis werden in frisches Wasser von 20-30 ° C mit dem Gewichtsanteil von 1000-2000 gemäß dem oben genannten Gewichtsanteil gegeben und für 4-6 Stunden eingeweicht, dann in einen Tontopf eingeschüttet, aufkochen lassen und für 20-30 Minuten weiter kochen, dann Lakritze und Lotosblatt zum obigen Verhältnis hinzufügen und weiter für 25-35 Minuten kochen, nach dem natürlichen Abkühlen wird der Rückstand des gekochten Medikamentes ausgefiltert, so die Flüssigkeit Nr. 1 vom Medikamente erhalten; Pseudostellaria, Fünfblättriger Ginseng und Hörner von Buffalo werden in frisches Wasser von 20-30 ° C mit dem Gewichtsanteil von 100 gegeben und für 4-6 Stunden eingeweicht, dann Pseudostellaria, Fünfblättriger Ginseng und Hörner von Buffalo ihre eingeweichte Flüssigkeit in den Tontopf gießen, aufkochen und dann 30 bis 50 Minuten kochen lassen, nach dem natürlichen Abkühlen wird der Rückstand des gekochten Medikamentes ausgefiltert, so die Flüssigkeit Nr. 2 vom Medikamente erhalten;
B) Flüssigkeit Nr. 1 und Nr. 2 vom Medikamente zusammen mischen, aufkochen und für 5 Minuten kochen lassen; nach dem natürlichen Abkühlen wird das so genannte Medikamente zur Behandlung von Leberaszites erhalten.

## Revendications

1. Le médicament à traiter l'ascite hépatique est **caractérisé par** les composants en poids ci-dessous :
Fleur de bambou 15-55, Épine de honeylocust chinoise 15-55, Corne de buffle 15-25, Caulis bambusae in taeniam 15-45, Radix angelicae pubescentis 15-25, Fructus schisandrae 15-25, Racine de puéraire 15-25, Herba hedyotis 15-75, Réglisse 5-15, Feuille de lotus 15-55, Semem cassiae 5-35, Ginseng caryophyllé 5-15, Ginseng américain 5-15, Eucommia ulmoides 5-35, Paeonia lactiflora 5-25, Radix sophorae flavescentis 5-35.

2. Le médicament à traiter l'ascite hépatique, selon ce qui a dit dans les revendications de brevet 1, est **caractérisé par** les composants en poids ci-dessous :
Fleur de bambou 35-55, Épine de honeylocust chinoise 35-55, Corne de buffle 15-25, Caulis bambusae in taeniam 15-25, Radix angelicae pubescentis 15-25, Fructus schisandrae 15-25, Racine de puéraire 15-25, Herba hedyotis 35-55, Réglisse 5-15, Feuille de lotus 35-55, Semem cassiae 5-15, Ginseng caryophyllé 5-15, Ginseng américain 5-15, Eucommia ulmoides 5-15, Paeonia lactiflora 5-15, Radix sophorae flavescentis 5-15.

3. Le médicament à traiter l'ascite hépatique, selon ce qui a dit dans les revendications de brevet 1,est **caractérisé par** les composants en poids ci-dessous :
Fleur de bambou 15-55, Épine de Honeylocust chinoise 15-25, Corne de buffle 15-25, Caulis Bambusae In Taeniam 35-45, Radix Angelicae Pubescentis 15-25, Fructus Schisandrae 15-25, Racine de puéraire 15-25, Herba Hedyotis 15-25, Réglisse 5-15, Feuille de lotus 5-15, Semem cassiae 25-35, Ginseng caryophyllé 5-15, Ginseng américain 5-15, Eucommia ulmoides 5-15, Paeonia lactiflora 5-25, Radix sophorae flavescentis 5-15.

4. La méthode de préparation du médicament à traiter l'ascite hépatique, selon ce qui a dit dans les revendications de brevet 1 ou 2 ou 3,est **caractérisé par** les étapes suivantes qui sont effectuées dans l'ordre:
A) Selon la ratio de poids ci-dessus, mettre la fleur de bambou, l' épine de Honeylocust chinoise, le semem cassiae , le fructus schisandrae, l'eucommia ulmoides, le paeonia lactiflora, le radix sophorae flavescentis, la racine de puéraire et l'herba hedyotis sous 1000-2000 part de poids dans l'eau à 20-30°C pour une macération pendant 4-6 heures, après avoir fait bouillir dans la casserole, continuer à bouillir pendant 20-30 minutes. Et puis, ajouter le réglisse et la feuille de lotus selon la ratio, continuer à bouillir pendant 25-35 minutes, après le refroidissement naturel, filtrer le résidu de décoction, et la solution 1 est obtenue;T remper respectivement le ginseng caryophyllé, le ginseng américain et la corne de buffle avec 100 parts de poids d'eau à 20-30 ° C pendant 4-6 heures, et puis mettre e ginseng caryophyllé, le ginseng américain, la corne de buffle et le liquide de trempage dans la casserole, après avoir fait bouillir, continuer à bouillir pendant 30-50 minutes. Après le refroidissement naturel, filtrer le résidu de décoction, et la solution 2 est obtenue,
B) Mélanger la solution 1 et la solution 2, chauffer à bouillir et le maintenir pendant 5 minutes, après le refroidissement naturel, on obtient le médicament mentionné à traiter l'ascite hépatique.
